# EUROPÄISCHE PATENTSCHRIFT

(11) **EP 3 488 409 B1**
(45) Veröffentlichungstag und Bekanntmachung des Hinweises auf die Patenterteilung: **19.03.2025**
(21) Anmeldenummer: 17752004.6
(22) Anmeldetag: 03.07.2017
(51) Int. Cl.: G06Q 10/08, G06Q 10/0832

(54) **DURCH BESTRAHLUNG STERILISIERBARE EINWEGSONDE**
DISPOSABLE PROBE THAT CAN BE STERILIZED BY IRRADIATION
SONDE À USAGE UNIQUE STÉRILISABLE PAR IRRADIATION

(30) Priorität: 20.07.2016 DE 102016113411
(43) Veröffentlichungstag der Anmeldung: 29.05.2019
(73) Patentinhaber: Sartorius Stedim Biotech GmbH, 37079 Göttingen (DE)
(72) Erfinder: GRIMM, Christian, 37308 Heilbad Heiligenstadt (DE); GAO, Wei, 37079 Göttingen (DE); LEUPOLD, Marco, 37079 Göttingen (DE)
(74) Vertreter: Novagraaf International SA
(86) Internationale Anmeldenummer: PCT/EP2017/066513
(87) Internationale Veröffentlichungsnummer: WO 2018/015135

(56) Entgegenhaltungen:
- WO-A1-2006/108500
- WO-A1-2006/108500
- US-A1- 2010 060 456
- US-A1- 2010 060 456
- US-A1- 2010 170 352
- US-A1- 2010 170 352
- US-A1- 2015 310 771
- US-A1- 2015 310 771
- US-B2- 8 963 684
- US-B2- 8 963 684

## Beschreibung

Die Erfindung betrifft eine Vorrichtungsanordnung mit einer durch Bestrahlung sterilisierbaren Einwegsonde für eine Einwegkomponente, die zur Verwendung in einem biopharmazeutischen Prozess vorgesehen ist.

Die Trends der letzten Jahre in der biopharmazeutischen Industrie weisen verstärkt in die Richtung der Verwendung von Einwegkomponenten (engl.: single use components, Abk.: SU-Komponenten). Diese werden nun auch nicht mehr nur im Bereich der Produkt- und Prozessentwicklung eingesetzt, sondern auch im Bereich der klinischen Prüfmusterherstellung (engl.: Clinical Trial Manufacturing, Abk.: CTM) für das Zulassungsverfahren und sogar in der kommerziellen "Guten Herstellungspraxis" (engl.: Good Manufacturing Practice, Abk.: GMP) bei der Produktion von Arzneimitteln.

Bei der kommerziellen Fertigung finden im Wesentlichen zwei verschiedene Ansätze der Nutzung von Einwegkomponenten Einzug. Zum einen setzen gerade bei neuen Fabriken, Standorten oder Unternehmen speziell in Asien die Betreiber auf einen vollen Single-Use-Ansatz, bei dem die ganze Herstellungskette nahezu ausschließlich mit Einwegkomponenten abgedeckt wird. Sind zum anderen klassische Geräte aus Edelstahl bereits vorhanden, sollen diese in der Regel aufgrund des hohen Anschaffungspreises auch genutzt werden. In diesem Fall kommt es zum Einsatz von so genannten Hybridanalagen aus bestehenden Edelstahlkomponenten und jeweils neu hinzukommenden Einwegkomponenten. Ein Beispiel dafür ist die Kultivierung von tierischen Zellen in einem Bioreaktor.

Insbesondere im Bereich der klinische Prüfmusterherstellung oder sogar im Umfeld der in den USA gebräuchlichen Methode der Überprüfung der momentanen Qualitätsstandards im Bereich Herstellung (engl.: Current Good Manufacturing Practice, Abk.: cGMP) ist es unerlässlich, dass die Integrität des Reaktionsgefäßes gegeben ist und somit sowohl ein Eindringen von Fremdorganismen als auch eine Austreten des Reaktionsmediums aus dem Reaktor verhindert werden kann. Bei Edelstahlreaktoren wird dies beispielsweise nach Reinigung, Installation und Dampfsterilisation durch Beaufschlagen eines Prüfdruckes und der Ermittlung des Druckabfalls über die Zeit als Messparameter sichergestellt. Diese Druckprüfung wird vom Betreiber der Anlage erfasst und geeignet dokumentiert. Bei Einwegbioreaktoren oder anderen aus Kunststoff hergestellten Systemen erfolgt dies direkt vor Benutzung auf ähnliche Weise. Direkt nach Installation des Behälters, wird das System über eine Schlauchverbindung aseptisch an eine Druckprüfungseinrichtung angeschlossen. Es sind Prüfgeräte erhältlich, mit denen sowohl die Druckprüfung als auch eine cGMP-konforme Dokumentation gewährleistet werden können, z.B. der "Sartocheck 4 plus Bag Tester" von Sartorius.

Da die Einweg-Systeme im Gegensatz zu Edelstahl-Systemen keine ortsfesten Anlagen sind, sind die Herstellung und der Transport der Behälter eine ernst zu nehmende Quelle von möglichen Leckagen. Um dem vorzubeugen, wird teilweise schon bei der Produktion von Einweg-Behältern eine Überprüfung der Behälterintegrität durchgeführt. Dazu werden beim Hersteller ähnliche Systeme verwendet, wie sie auch beim späteren Anwender vor der Benutzung zum Einsatz kommen. Wesentlicher Nachteil der bisherigen Methode ist, dass die Informationen zum Integritätstest nicht am Behälter selbst abgelegt sind und deshalb mühsam über die Losnummer (engl.: lot number) rückverfolgt werden müssen.

In einem anderen Zusammenhang ist aus der US 8 963 684 B2 ein gammasterilisierbares RFID-System bekannt, mit dem nicht vom Originalhersteller hergestellte Einweg-Bioprozess-Komponenten erkannt und daraufhin ein nicht autorisierter Betrieb verhindert werden können. Zu diesem Zweck wird ein FeRAM-Chip (engl.: ferroelectric random access memory chip) benutzt, um fehlerkorrigierbare Informationen auf einem RFID-Tag zu speichern, der an einer Einweg-Bioprozess-Komponente angebracht ist. Die Information bleibt in der Regel auch nach der Gammasterilisation des RFID-Tags und der Einweg-Bioprozess-Komponente im Speicherchip erhalten und kann gegebenenfalls korrigiert werden. Es ist auch ein Verfahren zur Authentifizierung der Einweg-Bioprozess-Komponente beschrieben, welches das Haftungsrisiko des Originalherstellers vermindern soll. Besteht eine Komponente, einen Authentifizierungstest nicht, wird eine Warnung an den Benutzer ausgegeben oder der Bioprozess wird angehalten. Auf diese Weise sollen sich minderwertige Fälschungen erkennen lassen und Benutzer in diesem Fall davon abgehalten werden, eine ungerechtfertigte Reklamation beim Originalhersteller geltend zu machen.

Die US 2015/0310771 A1 beschreibt beispielhaft einen RFID-Tag mit einem wiederbeschreibbaren Speicher in Verbindung mit einem "intelligenten Etikett", das mit einer Ware verbunden ist. Das intelligente Etikett kann über einen Sensor verfügen, um etwas über die physikalische oder chemische Umgebung zu erfassen, der die Ware während des Versandprozesses ausgesetzt war.

In der US 2010/0060456 A1 sind verschiedene Arten der Stromversorgung von Hochfrequenz-Kommunikationseinrichtungen genannt, wie etwa Kondensatoren oder Batterien. Ein solcher Energiespeicher kann Teil desselben Etiketts (tag) oder Labels sein wie die anderen Komponenten der Hochfrequenz-Kommunikationseinrichtung. Alternativ kann sich der Energiespeicher in einem von den anderen Komponenten des HF-Kommunikationsgeräts separaten Etikett oder Label befinden, wobei der Energiespeicher elektrisch mit den anderen Komponenten gekoppelt ist.

Aufgabe der Erfindung ist es, speziell die Dokumentation der Integritätsprüfung einer Einweg-Sensoreinheit, die in einem Bioprozess zum Einsatz kommt, zu verbessern und leichter verfügbar zu machen.

Gelöst wird diese Aufgabe durch eine Vorrichtungsanordnung mit einer Einwegsonde mit den Merkmalen des Anspruchs 1. Vorteilhafte und zweckmäßige Ausgestaltungen der erfindungsgemäßen Einwegsonde sind in den Unteransprüchen angegeben.

Die erfindungsgemäße Vorrichtungsanordnung umfasst eine Einwegsonde für eine Einwegkomponente, wobei die Einwegsonde zur Verwendung in einem biopharmazeutischen Prozess vorgesehen ist und sich in einer Verpackung befindet. Die Einwegsonde ist durch Bestrahlung sterilisierbar und umfasst wenigstes einen für den biopharmazeutischen Prozess relevanten Sensor, einen RFID-Tag und einen grundsätzlich wiederbeschreibbaren Speicher, in dem Daten bezüglich einer Integritätsprüfung der Einwegsonde abgelegt sind. Die Einwegsonde ist mit einer im RFID-Tag angeordneten Batterie ausgestattet, die dazu dient, die Elektronik der Einwegsonde mit Strom zu versorgen, sodass der Sensor der Einwegsonde bestimmungsgemäß genutzt und ggf. Messdaten in den Speicher geschrieben werden können. Die Vorrichtungsanordnung umfasst eine außerhalb der Einwegsonde an der Verpackung der Einwegsonde angebrachte Akkumulatoreinheit, die auf lösbare Weise elektrisch leitend mit einer Elektronik der Einwegsonde oder dem RFID-Tag verbunden ist und die Einwegsonde mit Energie versorgt, solange die Akkumulatoreinheit genug elektrische Leistung bereitstellen kann. Bei einem Öffnen der Verpackung, an der die Akkumulatoreinheit angebracht ist, wird die elektrische Leitung zwischen der Akkumulatoreinheit und der Einwegsonde unterbrochen und die interne Batterie des RFID-Tags übernimmt die weitere Energieversorgung, und der Zeitpunkt dieses Ereignisses in den Speicher geschrieben.

Der Begriff RFID-Tag ist nicht einschränkend zu verstehen und soll alle Typen von Transpondereinheiten, die drahtlos oder kontaktlos betreibbar und/oder auslesbar sind, und alle drahtlosen Übertragungsarten bzw. -standards einschließen, wie etwa NFC (Near Field Communication) etc.

Die Erfindung beruht auf der Erkenntnis, dass es elektronische Speicher gibt, die den Prozess einer Sterilisation der Einwegsonde durch Bestrahlung weitgehend unbeschadet überstehen. Dies eröffnet die Möglichkeit einer dynamisch gestalteten, am Produkt verbleibenden Dokumentation. Das bedeutet, dass nach einem ersten Ablegen von Daten im Speicher die Informationen nach der Bestrahlungssterilisation geändert und/oder ergänzt werden können. So lässt sich die Historie einer Einwegsonde sorgfältig dokumentieren und für Prüfzwecke leichter verfügbar machen.

Durch die Erfindung wird somit die Prozesssicherheit erhöht, da akkurate Daten über den Zustand bzw. die relevanten Eigenschaften der Einwegsonde sofort und an Ort und Stelle verfügbar sind. Dies führt einerseits zu weniger Reklamationen beim Hersteller der Einwegsonde und andererseits zu einer Verbesserung der Lieferkette.

Der Speicher, in dem die Daten vor und nach einer Bestrahlungssterilisation der Einwegsonde abgespeichert werden, ist vorzugsweise Bestandteil des RFID-Tags, sodass kein separates elektronisches Bauteil in die Einwegsonde integriert werden muss.

Als Speicher für die vorgesehenen Zwecke eignet sich ein FeRAM-Speicher. Es hat sich gezeigt, dass dieser Speichertyp weitestgehend resistent gegen die bei der Sterilisation von Einwegkomponenten verwendeten Strahlen(dosen) ist.

In dem Speicher sind vorteilhaft auch produktrelevante Daten abgelegt, insbesondere ein Produkt-Identifikationscode, ein Herstellungsdatum und/oder Kalibrierungsinformationen. Diese Daten können sich auf die Einwegsonde selbst und/oder auf die Einwegkomponente beziehen, für die die Einwegsonde bestimmt ist.

Dem Erfindungsgedanken folgend soll je nach Sondentyp wenigstens ein Messprinzip-spezifischer Qualitätsparameter festgelegt werden (z.B. bei einer elektronischen Temperatursonde der ohmsche Widerstand). Vor einer Bestrahlungssterilisation der Einwegsonde sollen dann - vorzugsweise unter reproduzierbaren Bedingungen - Werte dieses Messprinzip-spezifischen Qualitätsparameters ermittelt und in dem Speicher abgelegt werden.

Um eine aussagekräftige Prüfung der Integrität der Einwegsonde nach deren Bestrahlungssterilisation zu ermöglichen, werden nach der Bestrahlungssterilisation wiederum Werte dieses Messprinzip-spezifischen Qualitätsparameters ermittelt - vorzugsweise unter den gleichen Bedingungen - und in dem Speicher abgelegt. Somit ist ein Vergleich des Zustands bzw. der Eigenschaften der Einwegsonde vor und nach der Bestrahlungssterilisation möglich. Falls die Einwegsonde vorgegebene Qualitätsanforderungen nicht mehr erfüllt, wird sie ausgemustert und nicht ausgeliefert.

Falls die Einwegsonde durch die Bestrahlungssterilisation nicht gänzlich untauglich geworden ist, sondern nur eine nachvollziehbar verschobene oder verzerrte Messcharakteristik aufweist, die durch eine entsprechende Kalibrierung ausgeglichen werden kann, muss von einer Auslieferung der Einwegsonde nicht zwingend abgesehen werden. Vielmehr kann in diesem Fall in dem Speicher eine nach der Bestrahlungssterilisation der Einwegsonde korrigierte Spezifikation der Einwegsonde abgelegt werden, die beim bestimmungsgemäßen Einsatz der Einwegsonde dann berücksichtigt wird.

In einer vorteilhaften Ausgestaltung des Speichers der Einwegsonde weist dieser einen Schreibschutz auf, der nach seiner Aktivierung ein Löschen und Überschreiben der im Speicher abgelegten Daten verhindert.

Der Speicher kann auch vorteilhaft in einen freien, beschreibbaren Bereich und einen gesperrten Bereich, der nicht mehr beschreibbar ist, unterteilt sein. Im gesperrten Bereich können Herstellerdaten abgelegt sein, die nicht verändert werden sollen, während in den freien Speicher Informationen über die Integritätsprüfung der Einwegsonde und/oder Messdaten, die später während des bestimmungsgemäßen Gebrauchs der Einwegsonde ermittelt werden, geschrieben werden können.

Es hat sich gezeigt, dass bestimmte Batterietypen den Prozess einer Sterilisation der Einwegsonde durch Bestrahlung weitgehend unbeschadet überstehen. Basierend auf dieser überraschenden Erkenntnis verfügt der RFID-Tag gemäß der Erfindung über eine interne Batterie. Diese Batterie kann einerseits die für den bestimmungsgemäßen Gebrauch der Einwegsonde erforderliche Energie bereitstellen. Andererseits kann die Batterie ggf. auch die Dokumentation der Historie der Einwegsonde unterstützen, worauf später noch genauer eingegangen wird.

Die Einwegsonde kann beispielsweise eine auf dem ohmschen Widerstand basierte Temperatursonde sein, deren Messprinzip-spezifischer Qualitätsparameter der ohmsche Widerstand ist.

Ein anderes Beispiel für die Einwegsonde ist eine auf Voltammetrie basierte pH-Sonde, deren Messprinzip-spezifischer Qualitätsparameter ihr elektrisches Potential in einer definierten wässrigen Umgebung ist.

Die Einwegsonde der erfindungsgemäßen Vorrichtungsanordnung weist einen oder mehrere Sensoren zur Erfassung von bestimmten Ereignissen und/oder Umgebungsbedingungen auf. Die erfindungsgemäße Vorrichtungsanordnung umfasst ferner eine außerhalb der Einwegsonde an einer Verpackung angebrachte Akkumulatoreinheit, die auf lösbare Weise elektrisch leitend mit einer Elektronik der Einwegsonde oder dem RFID-Tag verbunden ist.

Die Akkumulatoreinheit ermöglicht es, die Historie der Einwegsonde über einen längeren Zeitraum zu dokumentieren, insbesondere im Falle einer langen Lagerung der Einwegsonde. Da die Akkumulatoreinheit wiederaufladbar ist, kann sie auch nach einem etwaigen, durch die Bestrahlungssterilisation der Einwegsonde bedingten Entladen zur Stromversorgung der Sensoren der Einwegsonde dienen.

Besonders bevorzugt ist eine Akkumulatoreinheit mit einer Einrichtung zur drahtlosen Aufladung durch eine externe Energiequelle. Die Akkumulatoreinheit kann in diesem Fall im verpackten Zustand der Einwegsonde aufgeladen werden, ohne dass eine Kabelverbindung oder dergleichen hergestellt werden muss.

Vorzugsweise ist die Akkumulatoreinheit an einer inneren Seite der Verpackung angebracht. Die Unterbrechung der lösbaren elektrischen Anbindung der Akkumulatoreinheit kann erkannt und als Zeitpunkt des Auspackens und der Benutzung der Einwegsonde dokumentiert werden.

Die erfindungsgemäße Vorrichtungsanordnung kann durch ein Schreibgerät ergänzt werden, das eingerichtet ist zum drahtlosen Schreiben von Daten in den Speicher der Einwegsonde sowie ein Lesegerät, das eingerichtet ist zum drahtlosen Auslesen von im Speicher abgelegten Daten. Selbstverständlich können das Schreibgerät und das Lesegerät als kombiniertes Schreib-/Lesegerät ausgebildet sein.

Gemäß der Erfindung wird bei einem Öffnen der Verpackung, an der die Akkumulatoreinheit angebracht ist, die elektrische Leitung zwischen der Akkumulatoreinheit und der Einwegsonde unterbrochen, und die interne Batterie des RFID-Tags übernimmt die weitere Energieversorgung, und der Zeitpunkt dieses Ereignisses wird in den Speicher geschrieben. Dieser Zeitpunkt soll das Auspacken und die Benutzung dokumentieren, oder dass ab diesem Zeitpunkt die Integrität der Einwegsonde seitens des Herstellers nicht mehr gewährleistet wird.

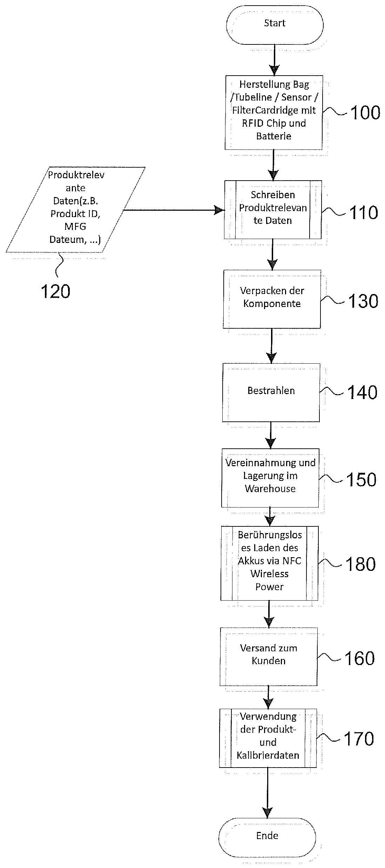

Weitere Merkmale und Vorteile der Erfindung ergeben sich aus der nachfolgenden Beschreibung und aus der beigefügten Zeichnung, auf die Bezug genommen wird. In der Zeichnung zeigt die einzige Figur beispielhaft den Lebenszyklus einer erfindungsgemäßen Einwegsonde.

Im Folgenden wird anhand des in der Figur gezeigten Flussdiagramms ein typischer Lebenslauf einer Einwegkomponente mit einer Einwegsonde beschrieben, beginnend mit dem Schritt 100 der Herstellung der Einwegkomponente, beispielsweise einem Behälter (Bag) zur Verwendung in einem Bioreaktor, und der Einwegsonde. Die Einwegsonde weist einen für einen biopharmazeutischen Prozess relevanten Sensor auf und ist zudem mit einem RFID-Tag (Transponder) versehen, der wiederum über einen grundsätzlich wiederbeschreibbaren Speicher verfügt, insbesondere vom Typ FeRAM.

In einem nächsten Schritt 110 werden mit einem geeigneten Schreibgerät produktrelevante Daten 120 drahtlos in den Speicher des RFID-Tags geschrieben. Die produktrelevanten Daten können einen Produkt-Identifikationscode, das Herstellungsdatum und weitere Angaben zur Einwegsonde umfassen, wie etwa Kalibrierungstabellen etc. Die produktrelevanten Daten können sich allgemein auf die Einwegsonde und/oder auf die Einwegkomponente beziehen.

Die Einwegkomponente mit der Einwegsonde wird, ggf. zusammen mit weiteren SU-Systemkomponenten, im Schritt 130 verpackt und im Schritt 140 als komplette Einheit, also einschließlich der Verpackung, durch Bestrahlung sterilisiert, insbesondere durch Gammabestrahlung. Ein FeRAM-Speicher übersteht in der Regel eine Sterilisation durch Bestrahlung, d.h. die im Speicher abgelegten Daten sind nach dem Sterilisationsprozess immer noch auslesbar.

Im Schritt 150 wird die sterilisierte Einheit vereinnahmt (bestandmäßige Erfassung) und eingelagert, bevor sie zu gegebener Zeit im Schritt 160 zu einem Kunden versandt wird.

Die einmalige Verwendung der Einwegkomponente mit der Einwegsonde beim Kunden ist durch den Schritt 170 dargestellt.

Der skizzierte Lebenslauf ist ein keiner Weise einschränkend zu verstehen und kann selbstverständlich weitere Schritte aufweisen.

Nach dem Schritt 140 des Bestrahlens und vor dem Schritt 160 des Versands zum Kunden kann seitens des Herstellers jederzeit die Integrität der Einwegsonde überprüft werden, wie unten noch näher erläutert wird. Außerdem können jederzeit die produktrelevanten Daten mit einem geeigneten Lesegerät aus dem Speicher des RFID-Tags ausgelesen und im Rahmen einer Bestands-, oder Qualitätsprüfung oder dergleichen ausgewertet werden. Schließlich können bei Bedarf noch zusätzliche Daten drahtlos in den Speicher des RFID-Tags geschrieben werden.

Im Folgenden werden die Durchführung, Dokumentation und Speicherung des Tests bzw. der Testergebnisse einer Einwegsonden-Integritätsprüfung genauer beschrieben. Grundprinzip der Prüfung ist ein Vergleich der Messprinzip-spezifischen Qualitätsparameter des jeweiligen Sensortyps der Einwegsonde vor und nach der Bestrahlungssterilisation. Für den jeweiligen Sensortyp werden hierfür vorab bestimmte sensorspezifische Qualitätsparameter ausgewählt, die für die Qualitätskontrolle nach der Bestrahlungssterilisation als geeignet angesehen werden. Außerdem wird eine tolerierbare Änderung jedes sensorspezifischen Qualitätsparameters gemäß den Eigenschaften des jeweiligen Sensortyps definiert.

In einem ersten Teil der Integritätsprüfung werden vor der Bestrahlungssterilisation die vorher festgelegten Messprinzip-spezifischen Qualitätsparameter der Einwegsonde über ein gängiges Verfahren zur Qualitätskontrolle ermittelt. Hierfür wird, soweit möglich, ein berührungsloses Verfahren gewählt (z.B. optisch, elektromagnetisch-induktiv oder radioaktiv). Die Qualitätskontrolle kann in diesem Fall noch durchgeführt werden, wenn die Einwegkomponente bereits verpackt ist. Die ermittelten Werte werden in den Speicher des RFID-Tags der Einwegsonde geschrieben.

Nach der Bestrahlungssterilisation werden in einem zweiten Teil der Integritätsprüfung die gleichen Qualitätsparameter erneut ermittelt, vorzugsweise wiederum berührungslos, damit die Einwegkomponente nicht ausgepackt werden muss. Diese Werte werden dann mit den im Speicher des RFID-Chips abgelegten Werten, die mit einem geeigneten Lesegerät ausgelesen werden verglichen, und es erfolgt eine Auswertung. Falls die durch die Bestrahlungssterilisation hervorgerufene Änderung der Qualitätsparameter innerhalb der definierten Toleranz liegt, werden die nach der Bestrahlungssterilisation ermittelten Qualitätsparameter als ein weiterer Datensatz im Speicher des RFID-Chips zur Lebenslaufüberwachung gespeichert. Die Einwegkomponente wird danach zur Kundenauslieferung freigegeben. Sollte die Änderung der Qualitätsparameter jedoch außerhalb der definierten Toleranz liegen, erhält die Einwegkomponente, an der die Einwegsonde angebracht ist, keine Freigabe zur Auslieferung.

Wie bereits erwähnt, kann der zweite Teil der Integritätsprüfung unmittelbar nach der Bestrahlungssterilisation und/oder während der Lagerung und/oder unmittelbar vor der geplanten Auslieferung an einen Kunden durchgeführt werden.

Des Weiteren können jeweils das Datum der Durchführung der Integritätsprüfung, der Einlagerung, die Lagerungsbedingungen (Ort, Temperatur etc.) als Informationen in den Speicher des RFID-Tags geschrieben werden. Aus diesen Daten lassen sich später Rückschlüsse auf den aktuellen Zustand ziehen und Aussagen darüber treffen, ob und ggf. wie lange die Einwegsonde für den vorgesehenen Einsatzzweck noch tauglich ist.

Nachfolgend werden zwei konkrete Beispiele für die Qualitätssicherung einer Einwegsonde beschrieben.

Im ersten Beispiel soll die Einwegsonde eine auf dem ohmschen Widerstand basierte Temperatursonde sein. Der Messprinzip-spezifische Qualitätsparameter ist hier also der ohmsche Widerstand, der vor und nach der Bestrahlungssterilisation bei möglichst gleicher Umgebungstemperatur ermittelt werden soll. Beispielsweise wird vor der Bestrahlungssterilisation bei einer konstanten Temperatur von 20°C ein Widerstand von 107,79 Ohm gemessen und mit den zugehörigen Messbedingungen in den Speicher des RFID-Tags geschrieben.

Nach der Bestrahlungssterilisation der Einwegkomponente mit der Temperatursonde wird bei der gleichen konstanten Temperatur der ohmsche Widerstand der Temperatursonde erneut ermittelt. Ist die Änderung des Widerstandswerts größer als ein vorher festgelegter Toleranzwert, z. B. +/- 0,3 Ohm, wird die Einwegkomponente ausgemustert, da sich der Nullpunkt und die Steilheit als Kalibrierparameter der Temperatursonde durch die Bestrahlungssterilisation so stark geändert haben, dass eine akzeptable Temperaturmessung beim Kunden nicht mehr gewährleistet ist.

Alternativ kann die Temperatur-Sonde weiteren Messungen bei anderen Temperaturen unterzogen werden, um den veränderten Nullpunkt und die veränderte Steilheit der Einwegsonde neu zu bestimmen und im Speicher des RFID-Tags abzulegen. Beispielsweise kann die Einwegkomponente mit der Temperatursonde auf konstant 5°C und konstant 45°C gebracht und jeweils der Widerstand der Temperatursonde bei diesen Temperaturen gemessen werden. Dadurch kann die Spezifikation der Temperatursonde korrigiert und die Temperatursonde mit korrigierter Spezifikation an einen Kunden ausgeliefert werden.

Um eine berührungslose Messung des ohmschen Widerstands zu ermöglichen, kann eine digitale Temperatursonde mit einer Spule zur induktiven Spannungsübertragung ausgerüstet werden. Aus der ermittelbaren verbrauchten elektrischen Leistung der Spannungsquelle wird auf den ohmschen Widerstand der Temperatursonde geschlossen.

Im zweiten Beispiel soll die Einwegsonde eine auf Voltammetrie basierte pH-Sonde sein. Der Messprinzip-spezifische Qualitätsparameter ist in diesem Fall das elektrische Potential der pH-Sonde in einer definierten wässrigen Umgebung, welches vor und nach der Bestrahlungssterilisation ermittelt werden soll. Zum besseren Verständnis sei angemerkt, dass die auf Voltammetrie basierte pH-Sonde in einer Kaliumchlorid-Lösung (KCI) eingelagert werden muss, um die Austrocknung des Elektrolyts in der Sonde zu vermeiden. Das im reinen Wasser gelöste KCI verursacht bei einer pH-Sonde ein elektrisches Potential von 0 mV. Deshalb kann das elektrische Potential unter der Voraussetzung der Lagerung mit einer solchen definierten KCI-Lösung als Qualitätsparameter für die pH-Sonde verwendet werden.

Beispielsweise wird vor der Bestrahlungssterilisation ein elektrische Potential der pH-Sonde von 0 mV gemessen und mit den zugehörigen Messbedingungen in den Speicher des RFID-Tags geschrieben. Nach der Bestrahlungssterilisation der Einwegkomponente mit der pH-Sonde wird bei gleichen Messbedingungen das elektrische Potential der pH-Sonde erneut ermittelt. Ist die Änderung des elektrischen Potentials größer als ein vorher festgelegter Toleranzwert, z. B. +/- 0,4 mV, wird die Einwegkomponente ausgemustert, da sich der Nullpunkt und die Steilheit als Kalibrierparameter der Temperatursonde durch die Bestrahlungssterilisation so stark geändert haben, dass eine akzeptable pH-Wert-Messung beim Kunden nicht mehr gewährleistet ist.

Ähnlich wie im ersten Beispiel besteht unter gewissen Voraussetzungen grundsätzlich die Möglichkeit, anhand des nach der Bestrahlungssterilisation ermittelten Messwerts und ggf. weiterer Messwerte die Spezifikation der pH-Sonde zu korrigieren und die pH-Sonde mit korrigierter Spezifikation an einen Kunden auszuliefern.

Um eine berührungslose Messung des elektrischen Potentials zu ermöglichen, können gängige elektromagnetisch-induktive Methoden eingesetzt werden.

Vor dem Versand zum Kunden kann im RFID-Tag ein Schreibschutz des Speichers aktiviert werden, um vor Manipulationen oder unbeabsichtigtem Löschen von Daten zu schützen, die im Speicher abgelegt sind. Dadurch ist gewährleistet, dass die korrekten Daten dauerhaft zur Verfügung stehen.

Der Kunde kann die produktrelevanten Daten und die Kalibrierinformationen bei Bedarf auslesen und entsprechend für seine Zwecke verwenden.

Die Einwegsonde ist zusätzlich mit einer im RFID-Tag angeordneten Batterie ausgestattet, die durch Bestrahlung mit Gammastrahlen oder anderen zur Sterilisation verwendeten Strahlen allenfalls teilweise beschädigt wird. Die Batterie dient insbesondere dazu, eine für den bestimmungsgemäßen Betrieb der Einwegsonde erforderliche Elektronik mit Strom zu versorgen, sodass der Sensor der Einwegsonde bestimmungsgemäß genutzt und ggf. Messdaten in den Speicher geschrieben werden können, wenn kein Strom von außen über Kabel oder kabellose Systeme verfügbar ist.

Darüber hinaus ist die Einwegsonde durch eine wiederaufladbare elektrische Energiequelle (Akkumulator) ergänzt. Eine über ein Kabel oder dergleichen elektrisch leitend mit der Elektronik der Einwegsonde bzw. deren RFID-Tag verbundene Akkumulatoreinheit ist z.B. an der inneren Seite einer Kartonage einer äußersten Umverpackung der Einwegkomponente angebracht und verfügt über eine Einrichtung zur drahtlosen Aufladung, wie etwa eine geeignete Nahfeldkommunikationsantenne (engl.: Near Field Communication, Abk.: NFC). Die Akkumulatoreinheit kann somit bei Bedarf von einer geeigneten externen Energiequelle berührungslos aufgeladen werden.

Die Verwendung einer solchen Akkumulatoreinheit erlaubt es, relevante Ereignisse und Umgebungsbedingungen in der Historie der Einwegsonde im Speicher des RFID-Tags über die Zeit, insbesondere vor einer Auslieferung an einen Kunden, zu dokumentieren. Dafür wird vorausgesetzt, dass die Einwegsonde über einen oder mehrere geeignete Sensoren zur Erfassung der Ereignisse und/oder Umgebungsbedingungen verfügt, wie etwa einen Temperatursensor, einen Feuchtigkeitssensor und/oder einen Strahlungssensor.

Bei der Fertigstellung oder zu einem späteren Zeitpunkt vor der Bestrahlungssterilisation wird die Akkumulatoreinheit geladen. Ab diesem Zeitpunkt können die relevanten Ereignisse, wie etwa die Bestrahlung selbst und die Strahlendosis sowie die Umgebungstemperatur und Raumfeuchtigkeit, in regelmäßigen oder unregelmäßigen Abständen ermittelt und in den Speicher der Einwegsonde geschrieben werden. Diese Daten können jederzeit mithilfe eines geeigneten Lesegeräts berührungslos ausgelesen werden.

Durch die Bestrahlungssterilisation kann die Akkumulatoreinheit jedoch eine Teilschädigung erleiden, was zu einer vollständigen oder teilweisen Entladung führt. Falls die Energie der Akkumulatoreinheit für die genannten Aufgaben nicht mehr ausreicht, wird dieser Zeitpunkt dokumentiert, d.h. in den Speicher geschrieben, und die interne Batterie des RFID-Tags übernimmt die weitere Stromversorgung.

Dank der Einrichtung zur drahtlosen Aufladung besteht grundsätzlich die Möglichkeit, den Akkumulator nach der Bestrahlungssterilisation bei Bedarf durch eine externe Energiequelle von außen berührungslos wieder aufzuladen, wie in der Figur durch den Schritt 180 dargestellt. Gegebenenfalls kann auch die Frequenz des Speicherns und des Auslesens der gespeicherten Sensordaten herabgesetzt oder angepasst werden, um die Abdeckung eines gewünschten Mindestzeitraums zu gewährleisten.

Die elektrisch leitende Verbindung zwischen dem RFID-Tag bzw. der Elektronik der Einwegsonde und der Akkumulatoreinheit ist derart gestaltet, dass beim Öffnen der Umverpackung, beim Entnehmen des Inhalts der Umverpackung (Einwegkomponente) oder bei einer definierten Erschütterung die Leitung unterbrochen wird. Die interne Batterie des RFID-Tags übernimmt dann die weitere Energieversorgung. Der Zeitpunkt der Unterbrechung wird dokumentiert, d.h. in den Speicher geschrieben, und kann als Hinweis darauf gedeutet werden, dass die Einwegsonde ausgepackt und benutzt wird oder dass die Integrität der Einwegsonde nicht mehr gegeben ist.

Im zweiten Beispiel soll die Einwegsonde eine auf Voltammetrie basierte pH-Sonde sein. Der Messprinzip-spezifische Qualitätsparameter ist in diesem Fall das elektrische Potential der pH-Sonde in einer definierten wässrigen Umgebung, welches vor und nach der Bestrahlungssterilisation ermittelt werden soll. Zum besseren Verständnis sei angemerkt, dass die auf Voltammetrie basierte pH-Sonde in einer Kaliumchlorid-Lösung (KCI) eingelagert werden muss, um die Austrocknung des Elektrolyts in der Sonde zu vermeiden. Das im reinen Wasser gelöste KCI verursacht bei einer pH-Sonde ein elektrisches Potential von 0 mV. Deshalb kann das elektrische Potential unter der Voraussetzung der Lagerung mit einer solchen definierten KCI-Lösung als Qualitätsparameter für die pH-Sonde verwendet werden.

Beispielsweise wird vor der Bestrahlungssterilisation ein elektrische Potential der pH-Sonde von 0 mV gemessen und mit den zugehörigen Messbedingungen in den Speicher des RFID-Tags geschrieben. Nach der Bestrahlungssterilisation der Einwegkomponente mit der pH-Sonde wird bei gleichen Messbedingungen das elektrische Potential der pH-Sonde erneut ermittelt. Ist die Änderung des elektrischen Potentials größer als ein vorher festgelegter Toleranzwert, z. B. +/- 0,4 mV, wird die Einwegkomponente ausgemustert, da sich der Nullpunkt und die Steilheit als Kalibrierparameter der Temperatursonde durch die Bestrahlungssterilisation so stark geändert haben, dass eine akzeptable pH-Wert-Messung beim Kunden nicht mehr gewährleistet ist.

Ähnlich wie im ersten Beispiel besteht unter gewissen Voraussetzungen grundsätzlich die Möglichkeit, anhand des nach der Bestrahlungssterilisation ermittelten Messwerts und ggf. weiterer Messwerte die Spezifikation der pH-Sonde zu korrigieren und die pH-Sonde mit korrigierter Spezifikation an einen Kunden auszuliefern.

Um eine berührungslose Messung des elektrischen Potentials zu ermöglichen, können gängige elektromagnetisch-induktive Methoden eingesetzt werden.

Vor dem Versand zum Kunden kann im RFID-Tag ein Schreibschutz des Speichers aktiviert werden, um vor Manipulationen oder unbeabsichtigtem Löschen von Daten zu schützen, die im Speicher abgelegt sind. Dadurch ist gewährleistet, dass die korrekten Daten dauerhaft zur Verfügung stehen.

Der Kunde kann die produktrelevanten Daten und die Kalibrierinformationen bei Bedarf auslesen und entsprechend für seine Zwecke verwenden.

Die Einwegsonde ist zusätzlich mit einer im RFID-Tag angeordneten Batterie ausgestattet, die durch Bestrahlung mit Gammastrahlen oder anderen zur Sterilisation verwendeten Strahlen allenfalls teilweise beschädigt wird. Die Batterie dient insbesondere dazu, eine für den bestimmungsgemäßen Betrieb der Einwegsonde erforderliche Elektronik mit Strom zu versorgen, sodass der Sensor der Einwegsonde bestimmungsgemäß genutzt und ggf. Messdaten in den Speicher geschrieben werden können, wenn kein Strom von außen über Kabel oder kabellose Systeme verfügbar ist.

Darüber hinaus ist die Einwegsonde durch eine wiederaufladbare elektrische Energiequelle (Akkumulator) ergänzt. Eine über ein Kabel oder dergleichen elektrisch leitend mit der Elektronik der Einwegsonde bzw. deren RFID-Tag verbundene Akkumulatoreinheit ist z.B. an der inneren Seite einer Kartonage einer äußersten Umverpackung der Einwegkomponente angebracht und verfügt über eine Einrichtung zur drahtlosen Aufladung, wie etwa eine geeignete Nahfeldkommunikationsantenne (engl.: Near Field Communication, Abk.: NFC). Die Akkumulatoreinheit kann somit bei Bedarf von einer geeigneten externen Energiequelle berührungslos aufgeladen werden.

Die Verwendung einer solchen Akkumulatoreinheit erlaubt es, relevante Ereignisse und Umgebungsbedingungen in der Historie der Einwegsonde im Speicher des RFID-Tags über die Zeit, insbesondere vor einer Auslieferung an einen Kunden, zu dokumentieren. Dafür wird vorausgesetzt, dass die Einwegsonde über einen oder mehrere geeignete Sensoren zur Erfassung der Ereignisse und/oder Umgebungsbedingungen verfügt, wie etwa einen Temperatursensor, einen Feuchtigkeitssensor und/oder einen Strahlungssensor.

Bei der Fertigstellung oder zu einem späteren Zeitpunkt vor der Bestrahlungssterilisation wird die Akkumulatoreinheit geladen. Ab diesem Zeitpunkt können die relevanten Ereignisse, wie etwa die Bestrahlung selbst und die Strahlendosis sowie die Umgebungstemperatur und Raumfeuchtigkeit, in regelmäßigen oder unregelmäßigen Abständen ermittelt und in den Speicher der Einwegsonde geschrieben werden. Diese Daten können jederzeit mithilfe eines geeigneten Lesegeräts berührungslos ausgelesen werden.

Durch die Bestrahlungssterilisation kann die Akkumulatoreinheit jedoch eine Teilschädigung erleiden, was zu einer vollständigen oder teilweisen Entladung führt. Falls die Energie der Akkumulatoreinheit für die genannten Aufgaben nicht mehr ausreicht, wird dieser Zeitpunkt dokumentiert, d.h. in den Speicher geschrieben, und die interne Batterie des RFID-Tags übernimmt die weitere Stromversorgung.

Dank der Einrichtung zur drahtlosen Aufladung besteht grundsätzlich die Möglichkeit, den Akkumulator nach der Bestrahlungssterilisation bei Bedarf durch eine externe Energiequelle von außen berührungslos wieder aufzuladen, wie in der Figur durch den Schritt 180 dargestellt. Gegebenenfalls kann auch die Frequenz des Speicherns und des Auslesens der gespeicherten Sensordaten herabgesetzt oder angepasst werden, um die Abdeckung eines gewünschten Mindestzeitraums zu gewährleisten.

Die elektrisch leitende Verbindung zwischen dem RFID-Tag bzw. der Elektronik der Einwegsonde und der Akkumulatoreinheit ist derart gestaltet, dass beim Öffnen der Umverpackung, beim Entnehmen des Inhalts der Umverpackung (Einwegkomponente) oder bei einer definierten Erschütterung die Leitung unterbrochen wird. Die interne Batterie des RFID-Tags übernimmt dann die weitere Energieversorgung. Der Zeitpunkt der Unterbrechung wird dokumentiert, d.h. in den Speicher geschrieben, und kann als Hinweis darauf gedeutet werden, dass die Einwegsonde ausgepackt und benutzt wird oder dass die Integrität der Einwegsonde nicht mehr gegeben ist.

## Patentansprüche

1. Vorrichtungsanordnung mit einer durch Bestrahlung sterilisierbaren Einwegsonde für eine Einwegkomponente, wobei die Einwegsonde zur Verwendung in einem biopharmazeutischen Prozess vorgesehen ist und sich in einer Verpackung befindet, und wobei die Einwegsonde wenigstens einen für den biopharmazeutischen Prozess relevanten Sensor, einen RFID-Tag und einen grundsätzlich wiederbeschreibbaren Speicher aufweist, in dem Daten bezüglich einer Integritätsprüfung der Einwegsonde abgelegt sind, **dadurch gekennzeichnet, dass** die Einwegsonde mit einer im RFID-Tag angeordneten Batterie ausgestattet ist, die dazu dient, die Elektronik der Einwegsonde mit Strom zu versorgen, sodass der Sensor der Einwegsonde bestimmungsgemäß genutzt und ggf. Messdaten in den Speicher geschrieben werden können, und dass die Vorrichtungsanordnung eine außerhalb der Einwegsonde an der Verpackung der Einwegsonde angebrachte Akkumulatoreinheit umfasst, die auf lösbare Weise elektrisch leitend mit einer Elektronik der Einwegsonde oder dem RFID-Tag verbunden ist und die Einwegsonde mit Energie versorgt, solange die Akkumulatoreinheit genug elektrische Leistung bereitstellen kann, wobei bei einem Öffnen der Verpackung, an der die Akkumulatoreinheit angebracht ist, die elektrische Leitung zwischen der Akkumulatoreinheit und der Einwegsonde unterbrochen wird und die interne Batterie des RFID-Tags die weitere Energieversorgung übernimmt, und der Zeitpunkt dieses Ereignisses in den Speicher geschrieben wird.

2. Vorrichtungsanordnung nach Anspruch 1, **dadurch gekennzeichnet, dass** der Speicher Bestandteil des RFID-Tags ist, und/oder dass der Speicher ein FeRAM-Speicher ist.

3. Vorrichtungsanordnung nach einem der vorhergehenden Ansprüche, **dadurch gekennzeichnet, dass** in dem Speicher produktrelevante Daten abgelegt sind, insbesondere ein Produkt-Identifikationscode, ein Herstellungsdatum und/oder Kalibrierungsinformationen.

4. Vorrichtungsanordnung nach einem der vorhergehenden Ansprüche, **dadurch gekennzeichnet, dass** in dem Speicher vor einer Bestrahlungssterilisation der Einwegsonde ermittelte Werte eines Messprinzip-spezifischen Qualitätsparameters der Einwegsonde abgelegt sind, und/oder dass in dem Speicher nach einer Bestrahlungssterilisation der Einwegsonde ermittelte Werte des Messprinzip-spezifischen Qualitätsparameters der Einwegsonde abgelegt sind, und/oder dass in dem Speicher eine nach einer Bestrahlungssterilisation der Einwegsonde korrigierte Spezifikation der Einwegsonde abgelegt ist.

5. Vorrichtungsanordnung nach einem der vorhergehenden Ansprüche, **gekennzeichnet durch** einen Schreibschutz, der nach Aktivierung ein Löschen und Überschreiben der im Speicher abgelegten Daten verhindert, und/oder dass der Speicher einen freien, beschreibbaren Bereich und einen gesperrten Bereich aufweist, der nicht mehr beschreibbar ist.

6. Vorrichtungsanordnung nach einem der vorhergehenden Ansprüche, **dadurch gekennzeichnet, dass** die Einwegsonde eine auf dem ohmschen Widerstand basierte Temperatursonde ist, deren Messprinzip-spezifischer Qualitätsparameter der ohmsche Widerstand ist, oder dass die Einwegsonde eine auf Voltammetrie basierte pH-Sonde ist, deren Messprinzip-spezifischer Qualitätsparameter ihr elektrisches Potential in einer definierten wässrigen Umgebung ist.

7. Vorrichtungsanordnung nach einem der vorhergehenden Ansprüche, **dadurch gekennzeichnet, dass** die Akkumulatoreinheit eine Einrichtung zur drahtlosen Aufladung durch eine externe Energiequelle aufweist, und/oder dass die Akkumulatoreinheit an einer inneren Seite der Verpackung angebracht ist.

8. Vorrichtungsanordnung nach einem der vorhergehenden Ansprüche, mit einem Schreibgerät, das eingerichtet ist zum drahtlosen Schreiben von Daten in den Speicher der Einwegsonde sowie einem Lesegerät, das eingerichtet ist zum drahtlosen Auslesen von im Speicher abgelegten Daten.

## Claims

1. Apparatus assembly, comprising a single-use probe for a single-use component, which single-use probe is sterilizable by irradiation, wherein the single-use probe is intended for use in a biopharmaceutical process and is located in a packaging, and wherein the single-use probe comprises at least one sensor relevant to the biopharmaceutical process, an RFID tag, and a memory which is rewritable in principle and in which data regarding an integrity check of the single-use probe are stored, **characterized in that** the single-use probe is equipped with a battery arranged in the RFID tag, which battery serves to supply power to the electronic unit of the single-use probe so that the sensor of the single-use probe can be used as intended and measurement data can be written to the memory when necessary, and **in that** the apparatus assembly comprises an accumulator unit which is mounted outside of the single-use probe on the packaging of the single-use probe and which is detachably and electrically conductively connected to an electronic unit of the single-use probe or to the RFID tag and supplies power to the single-use probe for as long as the accumulator unit can provide sufficient electrical power, wherein, upon opening the packaging on which the accumulator unit is mounted, the electrical lead between the accumulator unit and the single-use probe is broken and the internal battery of the RFID tag takes over the further power supply, and the time of this event is written to the memory.

2. Apparatus assembly according to claim 1, **characterized in that** the memory is part of the RFID tag, and/or **in that** the memory is a FeRAM memory.

3. Apparatus assembly according to any one of the preceding claims, **characterized in that** product-relevant data are stored in the memory, in particular a product identification code, a date of manufacture, and/or calibration information.

4. Apparatus assembly according to any one of the preceding claims, **characterized in that** values of a measurement-principle-specific quality parameter of the single-use probe that are determined before the single-use probe is sterilized by irradiation are stored in the memory, and/or **in that** values of the measurement-principle-specific quality parameter of the single-use probe that are determined after the single-use probe has been sterilized by irradiation are stored in the memory, and/or **in that** a specification of the single-use probe that has been corrected after the single-use probe has been sterilized by irradiation is stored in the memory.

5. Apparatus assembly according to any one of the preceding claims, **characterized by** a write protection which, after activation, prevents deletion and overwriting of the data stored in the memory, and/or in that the memory has a free, writable area and a locked area that is no longer writable.

6. Apparatus assembly according to any one of the preceding claims, **characterized in that** the single-use probe is a temperature probe based on ohmic resistance, the measurement-principle-specific quality parameter of which is the ohmic resistance, or **in that** the single-use probe is a pH probe based on voltammetry, the measurement-principle-specific quality parameter of which is the electric potential thereof in a defined aqueous environment.

7. Apparatus assembly according to any one of the preceding claims, **characterized in that** the accumulator unit comprises a device for wireless charging by an external power source, and/or **in that** the accumulator unit is mounted on an inner side of the packaging.

8. Apparatus assembly according to any one of the preceding claims, comprising a writing device which is designed to wirelessly write data to the memory of the single-use probe, and a reading device which is designed to wirelessly read data stored in the memory.

## Revendications

1. Ensemble de dispositif comprenant une sonde à usage unique stérilisable par irradiation pour un composant à usage unique, dans lequel la sonde à usage unique est destinée à être utilisée dans un processus biopharmaceutique et se trouve dans un emballage, et dans lequel la sonde à usage unique présente au moins un capteur important pour le processus biopharmaceutique, une étiquette RFID et une mémoire intrinsèquement réinscriptible, dans laquelle sont enregistrées des données relatives à un contrôle d'intégrité de la sonde à usage unique, **caractérisé en ce que** la sonde à usage unique est équipée d'une batterie disposée dans l'étiquette RFID, qui sert à alimenter l'électronique de la sonde à usage unique en courant, de sorte que le capteur de la sonde à usage unique est utilisé conformément à l'usage prévu et éventuellement des données de mesure peuvent être inscrites dans la mémoire, et que l'ensemble de dispositif comprend une unité d'accumulateur montée à l'extérieur de la sonde à usage unique sur l'emballage de la sonde à usage unique, qui est reliée de manière détachable et électro-conductrice à une électronique de la sonde à usage unique ou à l'étiquette RFID et alimente la sonde à usage unique en énergie, tant que l'unité d'accumulateur peut fournir suffisamment de puissance électrique, dans lequel lors d'une ouverture de l'emballage, sur lequel est montée l'unité d'accumulateur, la ligne électrique entre l'unité d'accumulateur et la sonde à usage unique est interrompue et la batterie interne de l'étiquette RFID prend en charge l'autre alimentation en énergie, et le moment de cet événement est inscrit dans la mémoire.

2. Ensemble de dispositif selon la revendication 1, **caractérisé en ce que** la mémoire fait partie de l'étiquette RFID, et/ou que la mémoire est une mémoire FeRAM.

3. Ensemble de dispositif selon l'une quelconque des revendications précédentes, **caractérisé en ce que** des données importantes pour le produit sont enregistrées dans la mémoire, en particulier un code d'identification de produit, une date de fabrication et/ou des informations d'étalonnage.

4. Ensemble de dispositif selon l'une quelconque des revendications précédentes, **caractérisé en ce que** des valeurs d'un paramètre de qualité spécifique au principe de mesure de la sonde à usage unique déterminées avant une stérilisation par irradiation de la sonde à usage unique sont enregistrées dans la mémoire, et/ou que des valeurs du paramètre de qualité spécifique au principe de mesure de la sonde à usage unique déterminées après une stérilisation par irradiation de la sonde à usage unique sont enregistrées dans la mémoire, et/ou qu'une spécification de la sonde à usage unique corrigée après une stérilisation par irradiation de la sonde à usage unique est enregistrée dans la mémoire.

5. Ensemble de dispositif selon l'une quelconque des revendications précédentes, **caractérisé par** une protection en écriture, qui après activation empêche une suppression et un écrasement des données enregistrées dans la mémoire, et/ou que la mémoire présente une zone libre, inscriptible et une zone verrouillée, qui n'est plus inscriptible.

6. Ensemble de dispositif selon l'une quelconque des revendications précédentes, **caractérisé en ce que** la sonde à usage unique est une sonde de température basée sur la résistance ohmique, dont le paramètre de qualité spécifique au principe de mesure est la résistance ohmique, ou que la sonde à usage unique est une sonde de pH basée sur la voltampérométrie, dont le paramètre de qualité spécifique au principe de mesure est son potentiel électrique dans un environnement aqueux défini.

7. Ensemble de dispositif selon l'une quelconque des revendications précédentes, **caractérisé en ce que** l'unité d'accumulateur présente un système pour la charge sans fil par une source d'énergie externe, et/ou que l'unité d'accumulateur est montée sur un côté intérieur de l'emballage.

8. Ensemble de dispositif selon l'une quelconque des revendications précédentes, comprenant un appareil d'inscription, qui est conçu pour écrire sans fil des données dans la mémoire de la sonde à usage unique ainsi qu'un appareil de lecture, qui est conçu pour la lecture sans fil de données enregistrées dans la mémoire.
